# FASCICULE DE BREVET EUROPEEN

(11) **EP 0 478 456 B1**
(45) Date de publication et mention de la délivrance du brevet: **18.01.1995**
(21) Numéro de dépôt: 91402567.1
(22) Date de dépôt: 26.09.1991
(51) Int. Cl.: A61K 9/12, A61K 47/34

(54) **Composition antifongique sous forme de spray sec**
Antimykotische Zusammensetzung in Trocken Aerosol Form
Antifungal composition in dry aerosol form

(30) Priorité: 26.09.1990 FR 9011886
(43) Date de publication de la demande: 01.04.1992
(73) Titulaire: SANDOZ LTD, 4002 Basel (CH)
(72) Inventeur: Laugier, Jean-Pierre, F-92160 Antony (FR); Ringenbach, François, F-92220 Bagneux (FR); Touzan, Philippe, F-31520 Ramonville Saint Agne (FR)

(56) Documents cités:
- EP-A- 0 298 271
- EP-A- 0 343 843
- FR-A- 2 113 920
- FR-A- 2 229 753
- US-A- 4 806 338
- 'THE MERCK INDEX , 11 EDITION'&& CO. ,RAHWAY , US no. 9086:"TERBINAFINE"

## Description

La présente invention a pour objet de nouvelles compositions dermatologiques sous forme de spray sec destinées au traitement pharmaceutique des mycoses et contenant un agent antifongique, associé à un polymère siliconé non cyclisé.

Les compositions sous forme de spray sec sont connues dans le domaine pharmaceutique en particulier pour traiter les mycoses suintantes. En effet, la forme poudre est tout à fait adaptée à ce genre de désordres cutanés et son application à l'aide d'un aérosol est pratique.

Il existe par exemple sur le marché des sprays secs poudres à base de nitrate d'éconazole ou de tolnaphtate.

Or, il existe pour ce type de spray des inconvénients liés à la concentration en principe actif qui peut-être restituée après pulvérisation par rapport à la concentration de ce même principe actif à l'intérieur de la bombe. De plus les pulvérisations ne restituent pas le même taux de principe actif au cours du temps.

Il est donc un but de la présente invention d'assurer que la concentration en principe actif dans le spray poudre, après pulvérisation, soit identique ou presque à celle dans la poudre contenue dans le réservoir aérosol, c'est-à-dire que le spray doit restituer, après pulvérisation, le même pourcentage d'actif que celui contenu dans la poudre du réservoir.

Il convient, en outre, d'assurer que chacune des pulvérisations successives restitue le même ou presque le même taux de principe actif, ce qui signifie que les pulvérisations doivent être parfaitement homogènes au cours du temps.

La solution à ces problèmes a jusqu'à maintenant consisté en un surdosage de la poudre en principe actif avant son conditionnement afin d'obtenir, en sortie de buse, la concentration souhaitée. Ceci entraîne cependant un surcroît du coût de production évident.

De plus, il est souvent nécessaire, afin d'éviter les problèmes techniques de colmatage, par exemple de la buse, de microniser la poudre avant son conditionnement en aérosol, ce qui représente une opération coûteuse, ou bien d'augmenter la pression du gaz propulseur au sein de l'aérosol, ce qui provoque, à l'application, un refroidissement important de la peau.

Or, il a maintenant été constaté de façon tout à fait inattendue que l'addition de certains polymères siliconés à la poudre contenue dans le spray sec permettait d'obtenir un dosage régulier du principe actif antifongique et également un titre sensiblement identique en cet actif dans la poudre contenue dans le boîtier aérosol et dans la poudre délivrée après pulvérisation.

L'association des polymères siliconés avec des sprays secs pharmaceutiques n'a pas jusqu'à maintenant été décrite, bien que dans le domaine cosmétique leur utilisation dans les aérosols antisudoraux ait été décrite par exemple dans le brevet américain n° 4.806.338. Toutefois, ce brevet vise des compositions antiperspirantes dont les composés actifs sont très souvent des composés hygroscopiques. Dans ce type de composition, les polymères siliconés jouent le rôle d'agent anti-hygroscopique en protégeant les particules solides de l'absorption précoce d'humidité et améliorent les qualités d'adhérence à la peau de la poudre après pulvérisation.

Parmi d'autres compositions cosmétiques aérosols qui contiennent des silicones, on peut citer les sprays "mouillés" à savoir ceux qui contiennent en outre un solvant organique. Dans ces produits, le silicone joue plusieurs rôles, par exemple le rôle de solvant (brevet britannique n° 1.167.173 et brevet français n° 2.113.920) ou encore le rôle de retardateur d'évaporation du solvant et ainsi améliore la qualité des gouttelettes du spray "mouillé" (brevet américain n° 4.152.416 et brevet européen n° 343.843).

La présente invention a par contre pour objet une composition pharmaceutique de spray sec contenant un agent antifongique de la famille des allylamines, de préférence la terbinafine et la naftifine et leurs sels notamment leur chlorhydrate.

La terbinafine est commercialisée par la Société SANDOZ sous le nom de LAMISIL (marque de commerce) et est décrite dans la demande de brevet européen n° 024.587.

Elle possède des propriétés antifongiques dans la mesure où elle inhibe la biosynthèse des stérols des membranes en intervenant vis-à-vis de l'enzyme squalène époxydase empêchant ainsi la synthèse de l'ergostérol indispensable aux champignons.

Cette particularité rend la terbinafine très efficace vis-à-vis des dermatophytes tels que par exemple le Trichophyton rubrum, l'Epidermophyton floccusum, le Trichophyton mentagrophytes, le Microsporum canis et le Trichophyton tonsurans.

Par ailleurs, la terbinafine comme la naftifine intervient également sur le métabolisme de l'ARN en inhibant la RNA-polymérase et intervient sur la synthèse des membranes en inhibant la chitine synthétase chez Saccharomyces cerevisiae.

Cette activité explique l'efficacité de la terbinafine sur différentes espèces d'Aspergillus, de Candida et de Fusarium.

Les agents antifongiques de la famille des allylamines ont notamment aucun caractère hygroscopique.

La présente invention a donc pour objet une composition antifongique sous forme de spray sec caractérisée par le fait qu'elle contient en association au moins un agent antifongique de la famille des allylamines, au moins un polymère siliconé non-cyclisé, au moins une charge minérale ou organique et au moins un propulseur.

De façon générale, la composition antifongique selon l'invention peut contenir en poids par rapport au poids total de la composition :
- de 0,01 à 0,25 % et de préférence de 0,04 à 0,15 % de l'agent antifongique,
- de 0,01 à 1,25 % et de préférence de 0,04 à 0,6 % du polymère siliconé non cyclisé,
- de 0,01 à 40 % et de préférence de 0,1 à 20 % de la charge minérale ou organique, et
- de 85 à 95 % du propulseur.

En effet, suite à des études, on a constaté que les polymères siliconés non cyclisés apportent, dans le cas des allylamines, une solution au problème de dosage équivalent entre la poudre dans l'aérosol et la poudre en sortie d'aérosol, sans qu'il soit nécessaire de surdoser en principe actif la poudre conditionnée en aérosol.

D'autre part, les polymères siliconés non cyclisés permettent le maintien au fur et à mesure de l'utilisation du spray, du titre en principe actif.

Parmi les polymères siliconés non cyclisés utilisables selon l'invention, on peut citer :
- les diméthicones (DM) de structure générale : 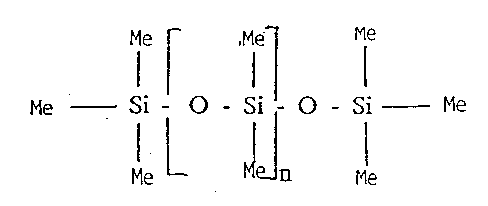 avec n compris entre 3 et 2 000, de préférence entre 20 et 1 000, tels que les produits vendus sous les dénominations BELSIL DM 0,65,35,100 et 350 par la Société WACKER ;
- les phényldiméthicones (PDM) de structure générale : 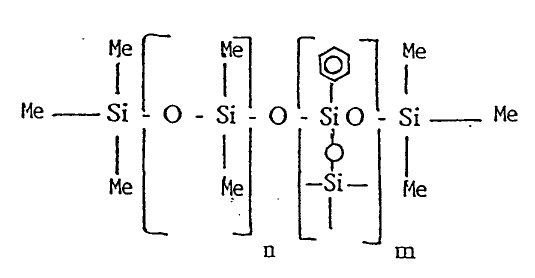 avec n et m compris entre 5 et 2 000, de préférence entre 20 et 1 000 tels que les produits vendus sous les dénominations BELSIL PDM 20, 200 et 1000 par la Société WACKER ;
- les phenyltriméthicones (PtM) de structure générale : 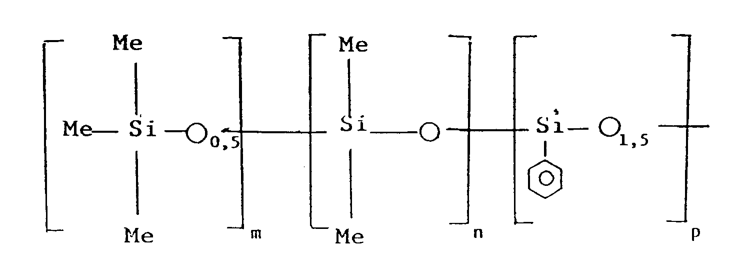 dans laquelle m, n et p sont indépendamment compris entre 2 et 2 000, de préférence entre 20 et 1 000, tels que les produits vendus par la Société GOLDSCHMIDT sous les dénominations ABIL AV 20, 200 et 2000.
- les diméthicones copolyols (DMC) de structure générale : 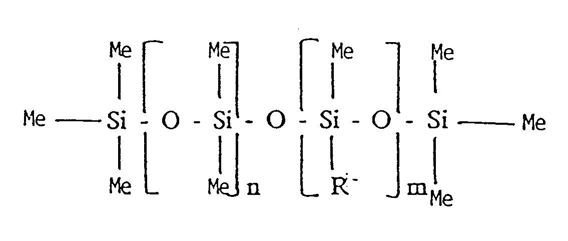 avec n et m compris entre 2 et 2 000, de préférence entre 20 et 1000 dans laquelle R est une chaîne copolyol ramifiées ou non de formule : 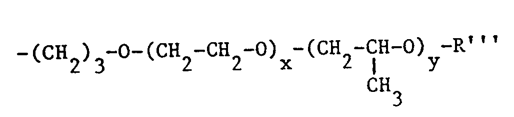 dans laquelle :
   x et y sont compris entre 1 et 20 et R''' est un radical alkyle à chaîne droite ou ramifiée ayant 10 à 18 atomes de carbone, tels que les produits vendus sous les dénominations BELSIL DMC 6031, 6032, 6033 et 6035 par la Société WACKER ;
- les stéaroxydiméthicones (SDM) de structure générale : 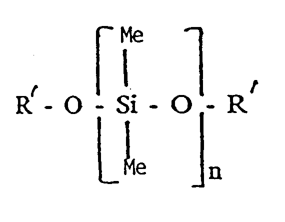 dans laquelle R' est une chaîne alkyle droite ou ramifiée en C₃ à C₂₀ et n est compris entre 3 et 2 000 telles que les produits vendus sous les dénominations BELSIL SDM 6021 et 6022 par la Société WACKER ; et
- les silicones aminofonctionnelles (SAM) de structure générale : 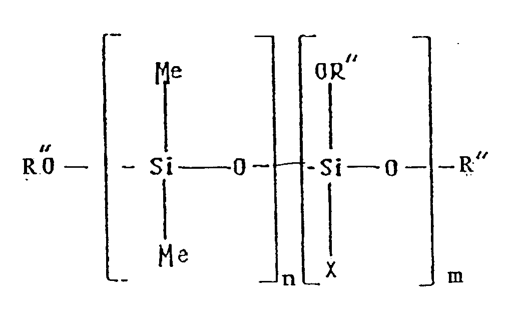 dans laquelle :
   n et m sont compris entre 2 et 2 000, de préférence entre 20 et 1 000 R'' représente indépendamment H, CH₃ ou Si(CH₃)₃ et
   X représente -(CH₂)₃-NHCH₂CH₂NH₂.

Parmi les silicones aminofonctionnelles on peut citer le produit vendu sous la dénomination d' "Amodiméthicone" de la Société WACKER.

On préfère employer les polymères siliconés non volatiles sous forme d'huile ayant une viscosité comprise entre 5 et 15 000 centistokes. (5 et 15 000 X 10⁻⁶m^{2/}S). Toutefois, la viscosité ne constitue pas un caractère critique pour la bonne mise en oeuvre de l'invention.

Les polymères siliconés sont utilisés à raison de 0,1 à 10 % en poids, et de préférence de 0,5 à 5 % par rapport au poids de la phase poudre.

L'agent antifongique est présent à une concentration comprise entre 0,1 et 2 % en poids et de préférence de 0,5 à 1,5 % par rapport au poids de la phase poudre.

Les propulseurs peuvent être des hydrocarbures aliphatiques liquéfiables tels que le propane, les butanes dont l'isobutane, le pentane, l'isopentane, le néopentane et leurs mélanges. On peut également employer des hydrocarbures halogénés tels que les chlorofluoroalkanes, dont le monochlorodifluoroéthane et le monochlorodifluorométhane et leurs mélanges.

Le rapport pondéral phase poudre-propulseur varie entre 1-8 et 1-12.

Parmi les charges minérales et organiques, on peut citer la silice colloïdale, l'oxyde de zinc, le talc, les tristéarates microcristallins et le stéarate de zinc.

De préférence, on utilise comme charge minérale la silice colloïdale anhydre, éventuellement en association avec au moins une autre charge minérale ou organique. La silice colloïdale facilite notamment la mise en suspension de la poudre avec le propulseur liquide avant chaque utilisation. Elle peut être utilisée à raison de 0,1 à 5% en poids et de préférence de 1 à 2 %, par rapport au poids de la phase poudre soit de 0,01 à 0,6 % et de préférence de 0,1 à 0,25% par rapport au poids total de la composition.

Enfin, des parfums et/ou conservateurs tels que l'alcool benzylique, peuvent être introduits dans les compositions selon l'invention.

La composition sous forme de spray poudre selon l'invention est particulièrement indiquée pour traiter les zones difficiles d'accès d'une part et les mycoses macérées d'autre part tels que : 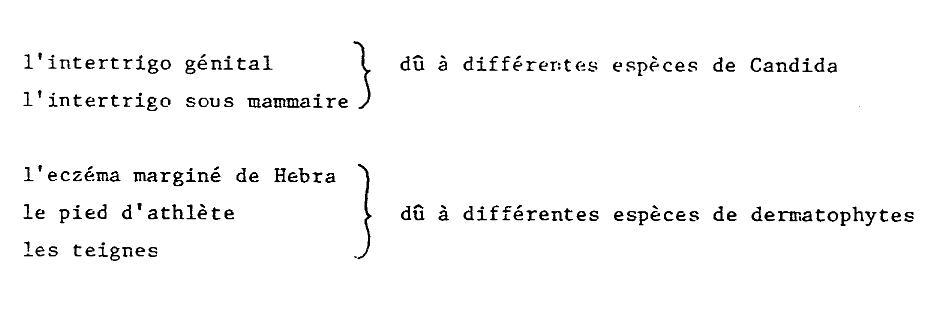
L'utilisation régulière de la composition sous forme de spray poudre selon l'invention lorsqu'elle contient 0,1 % d'un antifongique tel que la terbinafine se fera jusqu'à guérison complète des lésions à raison de 2 applications par jour.

Les exemples de compositions données ci-après illustrent l'invention de façon non limitative.

### EXEMPLE 1 : SPRAY SEC à 0,1 % de chlorhydrate de terbinafine

| | |
|---|---|
| Talc micronisé | 8,5 g |
| Silice colloïdale | 0,1 g |
| Tristéarate de glycérol microcristallin | 0,8 g |
| Diméthicone tel que le "BELSIL DM 100' de la Société WACKER | 0,5 g |
| Terbinafine HCl | 0,1 g |
| Propulseur : mélange de dichlorotétrafluoroéthane-dichlorodifluorométhane (40/60) qsp | 100,0 g |

On introduit dans un mélangeur classique pour poudre, le chlorhydrate de terbinafine, le talc micronisé, le tristéarate de glycérol puis la silice. Après mélange jusqu'à obtention d'un produit homogène, le polymère siliconé est ajouté progressivement.
Après agitation, on obtient une poudre qui n'adhère plus aux parois du mélangeur.
Cette poudre est alors disposée dans un bidon aérosol, qui est serti, puis dans lequel on introduit le mélange propulseur.

De la même manière que pour l'exemple 1 on a préparé les compositions des exemples 2 et 3.

### EXEMPLE 2 : SPRAY SEC à 0,1 % de chlorhydrate de terbinafine

| | |
|---|---|
| Oxyde de zinc | 4,0 g |
| Silice colloïdale | 0,2 g |
| Talc | 4,7 g |
| Phényldiméthicone tel que le "BELSIL PDM 200" de la Société WACKER | 1,0 g |
| Terbinafine HCl | 0,1 g |
| Propulseur : butane qsp | 100,0 g |

### EXEMPLE 3 : SPRAY SEC à 0,1 % de chlorhydrate de naftifine

| | |
|---|---|
| Oxyde de zinc | 4,3 g |
| Silice colloïdale | 0,1 g |
| Talc | 5,0 g |
| Stéaroxydiméthicone tel que le "BELSIL SDM 6021" de la Société WACKER | 0,6 g |
| Naftifine HCl | 0,1 g |
| Propulseur : mélange de monochlorodifluoroéthane et de monochlorodifluorométhane (60/40) qsp | 100,0 g |

### EXEMPLE 4 : SPRAY SEC à 0,1 % du chlorohydrate de terbinafine

| | |
|---|---|
| Oxyde de zinc | 4,1 g |
| Talc | 4,2 g |
| Silice colloïdale | 0,2 g |
| Diméthicone-copolyol tel que le "BELSIL DMC 6031" de la Société WACKER | 0,8 g |
| Terbinafine HCl | 0,1 g |
| Alcool benzylique | 0,3 g |
| Propulseur : butane qsp | 100,0 g |

Pour préparer ce spray, on procède de la façon suivante :
Dans un mélangeur à couteaux pour poudre, l'oxyde de zinc, le talc et la silice colloïdale sont introduits.
Après mélange, le chlorhydrate de terbinafine en solution dans l'alcool benzylique est ajouté à la préparation.
On mélange à nouveau jusqu'à obtention d'un produit homogène.
Au moment du conditionnement, le polymère siliconé est introduit directement dans les bidons, suivi de la phase poudre contenant le chlorhydrate de terbinafine.
Les bidons sont ensuite sertis puis mis sous pression par addition du propulseur.

### EXEMPLE 5 : SPRAY SEC à 0,1 % de chlorhydrate de naftifine

| | |
|---|---|
| Talc micronisé | 5,9 g |
| Stéarate de zinc | 3,1 g |
| Silice colloïdale | 0,5 g |
| Naftifine HCl | 0,1 g |
| Amodiméthicone vendu par la Société WACKER | 0,4 g |
| Propulseur : butane qsp | 100,0 g |

### EXEMPLE 6 : SPRAY SEC à 0,1% de chlorhydrate de terbinafine

| | |
|---|---|
| Talc micronisé | 9,0 g |
| Silice colloïdale | 0,4 g |
| Phényltriméthicone commercialisé sous la dénomination "ABIL AV 20" par la Société GOLDSCHMIDT | 0,5 g |
| Terbinafine HCl | 0,1 g |
| Propulseur : Isobutane qsp | 100,0 g |

## Revendications

1. Composition antifongique sous forme de spray sec, caractérisée par le fait qu'elle contient en association au moins un agent antifongique de la famille des allylamines, au moins un polymère siliconé non cyclisé, au moins une charge minérale ou organique et au moins un propulseur.

2. Composition antifongique selon la revendication 1, caractérisée par le fait qu'elle contient en poids par rapport au poids total de la composition :
- de 0,01 à 0,25 % et de préférence de 0,04 à 0,15 % de l'agent antifongique,
- de 0,01 à 1,25 % et de préférence de 0,04 à 0,6 % du polymère siliconé non cyclisé,
- de 0,01 à 40% et de préférence de 0,1 à 20 % de la charge minérale ou organique, et
- de 85 à 95 % du propulseur.

3. Composition selon la revendication 1 ou 2, caractérisée par le fait que l'agent antifongique de la famille des allylamines est choisi parmi la terbinafine et la Naftifine et leurs sels.

4. Composition selon l'une quelconque des revendications 1 à 3, caractérisée par le fait que le polymère siliconé non cyclisé est choisi parmi les diméthicones, les phényldiméthicones, les phényltriméthicones,les diméthicones-copolyols, les stéaroxydiméthicones et les silicones aminofonctionnelles.

5. Composition selon l'une quelconque des revendications précédentes, caractérisée par le fait que la charge minérale ou organique est choisie parmi la silice colloïdale, l'oxyde de zinc, le talc, les tristéarates microcristallins ou le stéarate de zinc.

6. Composition selon l'une quelconque des revendications précédentes, caractérisée par le fait qu'elle contient couple charge minérale de la silice colloïdale à raison de 0,01 à 0,6 % et de préférence de 0,1 à 0,25 % en poids.

7. Composition selon les revendications 1 à 6, caractérisée par le fait que le propulseur est choisi parmi les hydrocarbures aliphatiques liquéfiables tels que le propane, les butanes dont l'isobutane, le pentane, l'isopentane, le néopentane, les hydrocarbures halogénés, tels que les chlorofluoroalkanes dont le monochlorodifluoroéthane et le monochlorodifluorométhane et leurs mélanges.

## Claims

1. Antifungal composition in the form of a dry spray, characterized in that it contains, in combination, at least one antifungal agent of the allylamine family, at least one uncyclized silicone polymer, at least one inorganic or organic filler and at least one propellant.

2. Antifungal composition according to Claim 1, characterized in that it contains, by weight relative to the total weight of the composition:
- from 0.01 to 0.25%, and preferably from 0.04 to 0.15%, of the antifungal agent,
- from 0.01 to 1.25%, and preferably from 0.04 to 0.6%, of the uncyclized silicone polymer,
- from 0.01 to 40%, and preferably from 0.1 to 20%, of the inorganic or organic filler, and
- from 85 to 95% of the propellant.

3. Composition according to Claim 1 or 2, characterized in that the antifungal agent of the allylamine family is chosen from terbinafine and naftifine and their salts.

4. Composition according to any one of Claims 1 to 3, characterized in that the uncyclized silicone polymer is chosen from dimethicones, phenyl dimethicones, phenyl trimethicones, dimethicone copolyols, stearoxy dimethicones and amino-functional silicones.

5. Composition according to any of the preceding claims, characterized in that the inorganic or organic filler is chosen from colloidal silica, zinc oxide, talc, microcrystalline tristearates and zinc stearate.

6. Composition according to any one of the preceding claims, characterized in that it contains colloidal silica in the proportion of 0.01 to 0.6%, and preferably 0.1 to 0.25%, by weight, as inorganic filler.

7. Composition according to Claims 1 to 6, characterized in that the propellant is chosen from liquefiable aliphatic hydrocarbons such as propane, butanes including isobutane, pentane, isopentane, neopentane, halogenated hydrocarbons such as chlorofluoroalkanes including monochlorodifluoroethane and monochlorodifluoromethane, and mixtures thereof.

## Patentansprüche

1. Antimykotische Zubereitung in Form eines Trockensprays, dadurch gekennzeichnet, daß sie in der Zusammensetzung mindestens ein antimykotisches Mittel aus der Familie der Allylamine, mindestens ein acyclisches Silikonpolymer, mindestens einen mineralischen oder organischen Träger und mindestens ein Treibmittel enthält.

2. Antimykotische Zubereitung gemäß Anspruch 1, dadurch gekennzeichnet, daß sie in Gewichtsprozenten, bezogen auf das Gesamtgewicht der Zubereitung
- 0,01 bis 0,25 % und vorzugsweise von 0,04 bis 0,15 % antimykotischem Mittel,
- 0,01 bis 1,25 % und vorzugsweise von 0,04 bis 0,6 % an acyclischem Silikonpolymer,
- 0,01 bis 40 % und vorzugsweise von 0,1 bis 20 % an mineralischem oder organischem Träger und
- 85 bis 95 % Treibmittel
enthält.

3. Zubereitung gemäß Anspruch 1 oder 2, dadurch gekennzeichnet, daß das antimykotische Mittel aus der Familie der Allylamine aus Terbinafin und Naftifin sowie deren Salzen ausgewählt ist.

4. Zubereitung gemäß einem der Ansprüche 1 - 3, dadurch gekennzeichnet, daß das acyclische Silikonpolymer aus den Dimethikonen, Phenyldimethikonen, Phenyltrimethikonen, den Dimethikon-Copolyolen, Stearyloxydimethikonen und den Silikonen mit funktionellen Aminogruppen ausgewählt ist.

5. Zubereitung gemäß einem der vorstehenden Ansprüche, dadurch gekennzeichnet, daß der mineralische oder organische Träger ausgewählt ist aus kolloidalem Silizium, Zinkoxid, Talk, kristallinen Tristearaten oder Zinkstearat.

6. Zubereitung gemäß einem der vorstehenden Ansprüche, dadurch gekennzeichnet, daß sie als mineralischen Träger kolloide Kieselerde in einer Menge von 0,01 bis 0,6 Gew.-% und vorzugsweise von 0,1 bis 0,25 Gew.-% enthält.

7. Zubereitung gemäß den Ansprüchen 1 bis 6, dadurch gekennzeichnet, daß das Treibmittel aus verflüssigbaren, aliphatischen Kohlenwasserstoffen wie Propan, den Butanen, darunter Isobutan, Pentan, Isopentan, Neopentan, den Halogenkohlenwasserstoffen wie Fluorchloralkanen, darunter Monochlordifluorethan und Monochlordifluormethan, sowie deren Gemischen ausgewählt ist.
